# EUROPÄISCHE PATENTSCHRIFT

(11) **EP 0 557 498 B1**
(45) Veröffentlichungstag und Bekanntmachung des Hinweises auf die Patenterteilung: **24.01.2001**
(21) Anmeldenummer: 92919468.6
(22) Anmeldetag: 09.09.1992
(51) Int. Cl.: A61L 27/00, A61K 38/00, A61K 38/27

(54) **KNOCHENWACHSTUMSFÖRDERNDE ZUSAMMENSETZUNG**
BONE-GROWTH-STIMULATING COMPOSITION
COMPOSITION STIMULANT LA CROISSANCE OSSEUSE

(30) Priorität: 13.09.1991 DE 4130546
(43) Veröffentlichungstag der Anmeldung: 01.09.1993
(73) Patentinhaber: Baylink, David Jeston, Prof. Dr., Loma Linda CA 92357 (US); Ewers, Rolf, Prof. Dr. Dr., A-1160 Wien (AT); Kirsch, Axel, Dr., 70794 Filderstadt (DE)
(72) Erfinder: Baylink, David Jeston, Prof. Dr., Loma Linda CA 92357 (US); Ewers, Rolf, Prof. Dr. Dr., A-1160 Wien (AT); Kirsch, Axel, Dr., 70794 Filderstadt (DE)
(74) Vertreter: Goddar, Heinz J., Dr.
(86) Internationale Anmeldenummer: DE9200780
(87) Internationale Veröffentlichungsnummer: WO9305823

(56) Entgegenhaltungen:
- EP-A- 0 289 314
- EP-A- 0 312 208
- EP-A- 0 514 720
- WO-A-89/04646
- WO-A-89/10409
- WO-A-90/03810
- WO-A-90/04974
- WO-A-90/05522
- WO-A-90/12588
- US-A- 5 118 667

## Beschreibung

Die Erfindung betrifft die Verwendung einer knochenwachstumsfördernden Zusammensetzung zur Herstellung von Implanatmaterial.

Implantatmaterial, wie es heutzutage verwendet wird, besteht aus autologem Knochenmaterial, heterologem Knochenmaterial oder irgendeiner Form von Hydröxylapatit. Die Erklärung für die Verwendung eines solchen Implantatmaterials liegt darin, daß dieses Material die Heilung eines Knochendefektes schneller fördert, als wenn der Knochendefekt unbehandelt bleibt. Verschiedene Arten von Implantaten beschleunigen somit das Auffüllen des Knochendefekt. Wegen der Unmöglichkeit, ausreichend autologes Implantatmaterial zu beschaffen, sind heutzutage Hydroxylapatitformen von Implantatmaterial weitverbreitet. Das Problem mit Hydroxylapatitformen von Implantatmaterial besteht jedoch darin, daß sie nicht so schnell durch knöchernes Einwachsen eingebaut werden, wie es wünschenswert wäre, was die Behinderung des Patienten verlängert.

Aus der WO 88/00205 ist bekannt daß gewisse morphogene Knochenproteine, nämlich BMP-1, BMP-2 Klasse I, BMP-2 Klasse II, BMP-3 und Gemische derselben, in einem physiologisch akzektablen Vehikel bei der Erforschung und Behandlung von Knochen- und Periodontaldefekten nützlich sind. Außerdem schlägt diese Anmeldung vor, eine Matrix zuzusetzen, die in der Lage ist, die Zusammensetzung an den Ort des Knochendefekts zu bringen und eine Struktur für die Induzierung von Knochenbildung zur Verfügung zu stellen, z.B. Hydroxylapatit.

WO 90/04974 beschreibt ein Verfahren zur Behandlung von Zahnwurzelhauterkrankungen. Hierzu wird TGF-β in einer geeigneten Zusammensetzung verwendet, die, unter anderem, Fluorid als weiteres chemisches therapeutisches Agens enthält.

EP 0 514 720 A2 offenbart ein Verfahren zur Stimulierung von Knochenneubildung durch Verabreichung einer Kombination eines Wachstumsfaktors, wie beispielsweise TGF-β oder BMP, zusammen mit einem Knochenresorptionsinhibitor, wie beispielsweise Natriumfluorid.

Der Erfindung liegt die Aufgabe zugrunde, eine neue Verwendung einer Knochenwachstumsfördernden Zusammensetzung bereitzustellen.

Erfindungsgemäß wird diese Aufgabe gelöst durch die Verwendung nach Anspruch 1. Weitere Ausführungsformen ergeben nich aus den Unteransprüchen.

In einem ersten Ansatz, die Steigerung der Vermehrung der Knochenzellen zu erklären, vermuten wir, daß der Verstärkungseffekt von Fluorid-, Vanadat- oder Molybdatverbindungen auf deren hemmender Wirkung auf Phosphotyrosylproteinphosphatase beruht. Dieses Enzym bewirkt die Dephosphorylierung von Phosphoproteinen, induziert durch mitogene Signale, wodurch diese mitogenen Signale inaktiviert werden. Durch Hemmung dieser Dephosphorylierung fördert man dann das mitogenen Signal, das vom Wachstumsfaktor erzeugt wird.

Die in den Zusammensetzungen verwendeten Wachstumsfaktoren können durch eine Reihe von verschiedenen Techniken hergestellt werden.

Erstens schlägt die vorliegende Erfindung vor, daß wenigstens einer von ihnen aus natürlichem, vorzugsweise menschlichem Knochenmaterial gewonnen wird, wie z.B. IGF-II, von Mojan et al., Biochem. Biophys. Acta 884, 234 (1986) beschrieben. Alternativ dazu können die Wachstumsfaktoren auch aus Knochenzellen isoliert werden, die in Kultur gezüchtet worden sind.

Der zweite Weg für die Gewinnung der Wachstumsfaktoren ist die Isolierung aus Serum, einschließlich Human-, Rinder-, Schafs-, Schweine- oder Pferdeserum, unter Verwendung herkömmlicher Techniken.

Die dritte (und möglicherweise letztendlich die bevorzugte) Herstellungsmethode umfaßt jedoch rekombinante DNA-Techniken, d.h. die Gewinnung der Wachstumsfaktoren oder der biologisch aktiven Mutanten oder Fragmente derselben als rekombinante Verbindung aus einem gentechnologischen Verfahren. Einige dieser Techniken sind z.B. für IGF-II in obengenannter europäischer Patentanmeldung 0 289 314 und für BMP in obengenannter Internationalen Patentanmeldung WO 88/00205 beschrieben.

Schließlich kann sich die verwendete Zusammensetzung durch einen Gehalt an einem mitogenen Knochenextrakt auszeichnen. Die Herstellung eines solchen wirksamen Knochenextrakts, der einen oder mehrere der genannten Wachstumsfaktoren enthält, kann mit Hilfe herkömmlicher Techniken durchgeführt werden.

Die erfindungsgemäße Verwendung fördert das optimale Einwachsen von Knochen in das Implantatmaterial, wobei letztendlich ein schnelles Auffüllen von z.B. einem Knochendefekt verwirklicht werden kann. Ein Weg, solch ein Implantatmaterial herzustellen besteht darin, eine der genannten Kombinationen aus einem oder mehreren der genannten Wachstumsfaktoren oder biologisch aktiven Mutanten oder Fragmenten derselben oder aus einem entsprechenden Knochenextrakt kombiniert mit einem der genannten Salze aus der Gruppe der Vanadate, Molybdate und Fluoride, auf poröse Hydroxylapatit-Körner als Applikationsmaterial aufzubringen. Es hat sich gezeigt, daß ein chirurgisch erzeugter Knochendefekt mit solchen Hydroxylapatit-Körnern, verglichen mit Hydroxylapatit allein, schneller und vollständiger ausheilt.

Die Erfindung wird nunmehr anhand der folgenden Beispiele weiter veranschaulicht:

### BEISPIEL

Für die Anwendung der vorliegenden Erfindung wurden 750 µm Hydroxylapatit mit 250 µm Natriumfluorid und 200 ng FGF, TGF-β, IGF-II, PDGF, Knochenextrakt mit entsprechender Aktivität oder BMP-Aktivität oder Gemischen derselben komplexiert, um ein Implantat herzustellen. Solche Implantate erwiesen sich als nützlich für die Behandlung von Knochendefekten, wie bei unregelmäßigen Brüchen, bei allen Defekten aus Trauma oder chirurgischem Eingriff, in Verbindung mit Krebs, bei allen Defekten aus Infektion (Osteomylitis), bei alveolaren Defekten und bei Periodontalerkrankungen und kongenitalen Anomalien. Bei allen getesteten Implantaten war das Einwachsen von Knochen signifikant erhöht.

## Patentansprüche

1. Verwendung einer knochenwachstumfördernden Zusammensetzung mit einem Gehalt an wenigstens einer Substanz aus der Gruppe, die aus den Wachstumsfaktoren FGF, TGF-β, IGF-II, PDGF und BMP und Knochenextrakten mit entsprechender Aktivität besteht, sowie einem Gehalt an wenigstens einem Phosphotyrosylproteinphosphatase-Inhibitor in Form eines Salzes aus der Gruppe, die aus Vanadaten, Molybdaten und Fluoriden besteht, ausgenommen die Kombination von IGF-II mit Fluorid und Orthovanadat und die Kombination von TGF-β mit Fluorid sowie BMP mit Fluorid, in Kombination mit einem geeigneten Applikationsmaterial, zur Herstellung von Implantatmaterial.

2. Verwendung nach Anspruch 1, dadurch gekennzeichnet, daß das Applikationsmaterial Hydroxylapatit ist.

3. Verwendung nach Anspruch 1, dadurch gekennzeichnet, daß das Applikationsmaterial aus Algen-, Korallen- und Muschelderivat (en) besteht.

4. Verwendung nach einem der Ansprüche 1 bis 3, dadurch gekennzeichnet, daß das Applikationsmaterial porös ist.

5. Verwendung nach Anspruch 4, dadurch gekennzeichnet, daß das poröse Applikationsmaterial eine regulierbare Porengröße besitzt.

6. Verwendung nach einem der vorangehenden Ansprüche, dadurch gekennzeichnet, daß die Zusammensetzung einen Gehalt an wenigstens zwei Substanzen aus der Gruppe, die aus den Wachstumsfaktoren FGF, TGF-β, IGF-II, PDGF und BMP und Knochenextrakten mit entsprechender Aktivität besteht.

7. Verwendung nach Anspruch 6, dadurch gekennzeichnet, daß die Zusammensetzung in Kombination vier Wachstumsfaktoren enthält.

8. Verwendung nach einem der vorangehenden Ansprüche, dadurch gekennzeichnet, daß das mit einem Vanadat-, Molybdat- oder Fluorid-Ion verknüpfte Kation ausgewählt ist aus der Gruppe, die aus Natrium, Kalium, Calcium, Magnesium und Ammonium besteht.

9. Verwendung nach einem der vorangehenden Ansprüche, dadurch gekennzeichnet, daß wenigstens einer der Wachstumsfaktoren aus natürlichem Knochenmaterial gewonnen ist.

10. Verwendung nach Anspruch 9, dadurch gekennzeichnet, daß wenigstens einer der Wachstumsfaktoren aus menschlichem Knochenmaterial gewonnen ist.

11. Verwendung nach einem der vorangehenden Ansprüche, dadurch gekennzeichnet, daß wenigstens einer der Wachstumsfaktoren aus Serum, einschließlich Human-, Rinder-, Schafs-, Schweine- oder Pferdeserum, gewonnen ist.

12. Verwendung nach einem der vorangehenden Ansprüche, dadurch gekennzeichnet, daß wenigstens einer der Wachstumsfaktoren als rekombinante Verbindung aus einem gentechnologischen Verfahren gewonnen ist.

13. Verwendung nach einem der vorangehenden Ansprüche, dadurch gekennzeichnet, daß die Zusammensetzung einen Gehalt an einem mitogenen Knochenextrakt aufweist.

14. Verwendung nach einem der vorangehenden Ansprüche, dadurch gekennzeichnet, daß poröse Hydroxylapatit-Körner als Applikationsmaterial mit den übrigen Bestandteilen der Zusammensetzung überzogen sind.

## Claims

1. Use of a bone growth-promoting composition containing at least one substance from the group comprising the growth factors FGF, TGF-β, IGF-II, PDGF and BMP and bone extracts having corresponding activity and also containing at least one phosphotyrosyl protein phosphatase inhibitor in the form of a salt from the group comprising vanadates, molybdates and fluorides except for IGF-II in combination with fluoride and orthovanadate or TGF-β in combination with fluoride or BMP with fluoride, in combination with a suitable application material for producing implant material.

2. Use according to claim 1, characterised in that the application material is hydroxyl apatite.

3. Use according to claim 1, characterised in that the application material comprises one or more algae, coral and/or mussel derivatives.

4. Use according to any of claims 1 to 3, characterised in that the application material is porous.

5. Use according to claim 4, characterised in that the porous application material has an adjustable pore size.

6. Use according to any of the preceding claims, characterised in that the composition contains at least two substances from the group comprising the growth factors FGF, TGF-β, IGF-II, PDGF and BMP and bone extracts having similar activity.

7. Use according to claim 6, characterised in that the combination contains four growth factors in combination.

8. Use according to any of the preceding claims, characterised in that the cation linked to a vanadate, molybdate or fluoride ion is from the group comprising sodium, potassium, calcium, magnesium and ammonium.

9. Use according to any of the preceding claims, characterised in that at least one of the growth factors is obtained from natural bone material.

10. Use according to claim 9, characterised in that at least one of the growth factors is obtained from human bone material.

11. Use according to any of the preceding claims, characterised in that at least one of the growth factors is obtained from serum, including human, bovine, sheep, pig or horse serum.

12. Use according to any of the preceding claims, characterised in that at least one of the growth factors is obtained in the form of a recombinant compound by genetic engineering.

13. Use according to any of the preceding claims, characterised in that the composition contains a mitogenic bone extract.

14. Use according to any of the preceding claims, characterised in that application material in the form of porous hydroxyl apatite grains is coated with the other constituents of the composition.

## Revendications

1. Utilisation d'une composition stimulant la croissance osseuse, présentant une teneur en au moins une substance issue du groupe constitué des facteurs de croissance FGF, TGF-β, IGF-II, PDGF et BMP, et d'extraits d'os d'activité correspondante, ainsi qu'une teneur en au moins un inhibiteur de phosphotyrosylprotéinephosphatase, sous la forme d'un sel issu du groupe des vanadates, molybdates et fluorures, à l'exception de la combinaison de IGF-II avec du fluorure et de l'orthovanadate, et la combinaison de TGF-β avec du fluorure, ainsi que BMP avec du fluorure, en combinaison avec un matériau d'application approprié pour la préparation de matériau d'implant.

2. Utilisation selon la revendication 1, caractérisée en ce que le matériau d'application est de l'hydroxylapatite.

3. Utilisation selon la revendication 1, caractérisée en ce que le matériau d'application est constitué de dérivé(s) algène, koralène et de coquille.

4. Utilisation selon l'une des revendications 1 à 3, caractérisée en ce que le matériau d'application est poreux.

5. Utilisation selon la revendication 4, caractérisée en ce que le matériau d'application poreux comporte une taille de pores pouvant être régulée.

6. Utilisation selon l'une des revendications précédentes, caractérisée en ce que la composition présente une teneur en au moins deux substances issues du groupe constitué des facteurs de croissance que sont FGF, TGF-β, IGF-II, PDGF et BMP et d'extraits osseux d'activité correspondante.

7. Utilisation selon la revendication 6, caractérisée en ce que la composition contient, en combinaison, quatre facteurs de croissance.

8. Utilisation selon l'une des revendications précédentes, caractérisée en ce que le cation combiné à un ion vanadate, molybdate ou fluorure est sélectionné dans le groupe constitué du sodium, potassium, calcium, magnésium et ammonium.

9. Utilisation selon l'une des revendications précédentes, caractérisée en ce qu'au moins l'un des facteurs de croissance est obtenu à partir de matériau osseux naturel.

10. Utilisation selon la revendication 9, caractérisé en ce qu'au moins l'un des facteurs de croissance est obtenu à partir de matériau osseux humain.

11. Utilisation selon l'une des revendications précédentes, caractérisée en ce qu'au moins l'un des facteurs de croissance est obtenu à partir de sérum, y compris de sérum humain, bovin, ovin, porcin ou chevalin.

12. Utilisation selon l'une des revendications précédentes, caractérisée en ce qu'au moins l'un des facteurs de croissance est obtenu à partir de liaisons recombinantes, à partir d'un procédé à technologie génétique.

13. Utilisation selon l'une des revendications précédentes, caractérisée en ce que la composition présente une teneur en extrait humain mitogène.

14. Utilisation selon l'une des revendications précédentes, caractérisée en ce que les grains poreux d'hydroxylapatite, utilisés comme matériau d'application, sont revêtus des composants usuels de la composition.
